(19) Europäisches Patentamt
European Patent Office
Office européen des brevets

(11) Publication number: 0 318 330 A2

# (12) EUROPEAN PATENT APPLICATION

(21) Application number: 88311240.1

(22) Date of filing: 28.11.88

(51) Int. Cl.⁴: **A 61 K 37/02**

(30) Priority: 26.11.87 JP 298759/87

(43) Date of publication of application: 31.05.89 Bulletin 89/22

(84) Designated Contracting States: AT BE CH DE ES FR GB GR IT LI LU NL SE

(71) Applicant: YAMANOUCHI PHARMACEUTICAL CO. LTD.
No. 3-11 Nihonbashi-Honcho, 2-chome Chuo-ku
Tokyo (JP)

(72) Inventor: Asano, Masaharu
1-10-A206, Aioi-cho
Itabashi-ku Tokyo 174 (JP)

Uchida, Wataru
3-41-16-502, Hikawadai
Nerima-ku Tokyo 174 (JP)

Shibasaki, Kumiko
3-27-B1107, Nakadai
Itabashi-ku Tokyo 174 (JP)

(74) Representative: Geering, Keith Edwin et al
REDDIE & GROSE 16 Theobalds Road
London WC1X 8PL (GB)

## (54) Therapeutic agents for ischemic heart diseases.

(57) The use for the preparation of therapeutic agent for ischemic heart diseases of an effective component selected from glutathione monoalky esters of formula (I) and salts thereof

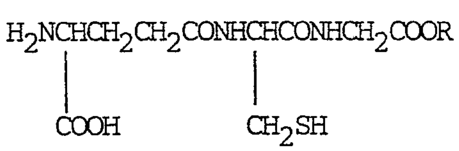

(I)

wherein R represents an alkyl group, preferably of 1-10 carbon atoms - e.g. an isopropyl group.

EP 0 318 330 A2

## Description

### Therapeutic agents for ischemic heart diseases

The present invention relates to therapeutic agents for ischemic heart diseases comprising an effective component selected from glutathione monoalkyl esters of formula (I) and salts thereof:

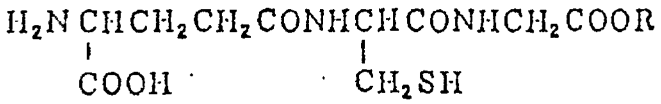

(I)

wherein R represents an alkyl group, preferably of 1 to 10 carbon atoms.

Recently, in the field of diseases of the circulary system, the number of patients suffering from ischemic heart disease such as angina pectoris and myocardial infarction has been increasing. Among these, myocardial infarction is very serious and various therapies have been studied. Most recently, as therapeutics for myocardial infarction, PTCA and PTCR have been widely used in clinical therapy and their effectiveness has been recognized. Although these therapeutics aid healing of the cardiac muscle from ischemic disorders, it has been disclosed that new disorders, for example, dangerous ventricular arrhythmia such as ventriclular fibrillation due to occluded coronary artery reperfusion, hemorrhagic infarction or no-reflow (poor reperfusion) phenomena etc., appear irreversibly (See Braunwald and Kloner, J. Clin. Invest., 76, 1713-1719, 1985). There have been no satisfactory drugs for the treatment of these new disorders.

We have sought to develop therapeutic agents for ischemic heart disease, especially drugs for controlling disorders which accompany occluded coronary artery reperfusion, and have found that the glutathione monoalkyl esters show marked efficacy for treatment of these diseases.

Our EP-A-0249401 and EP-A-0257992 disclose that glutathione monoalkyl esters are useful as anti-anemic agents and as prophylactic agents for cerebral ischemia.

The present invention directed to therapeutic agents for ischemic heart diseases is quite distinct from those in the kind of disease concerned and the range of application for use.

In formula (I) above, the alkyl group is straight or branched, preferably of 1 to 10 carbon atom. Specific examples include methyl, ethyl, propyl, isopropyl, butyl, isobutyl tert-butyl pentyl isopentyl, neopentyl, tert-pentyl, 1-methylbutyl, hexyl, isohexyl, 2-methylpentyl, 1-ethylbutyl, heptyl, octyl, nonyl and decyl groups, etc. Compounds (I) or salts thereof can be produced, for example, by the following process:

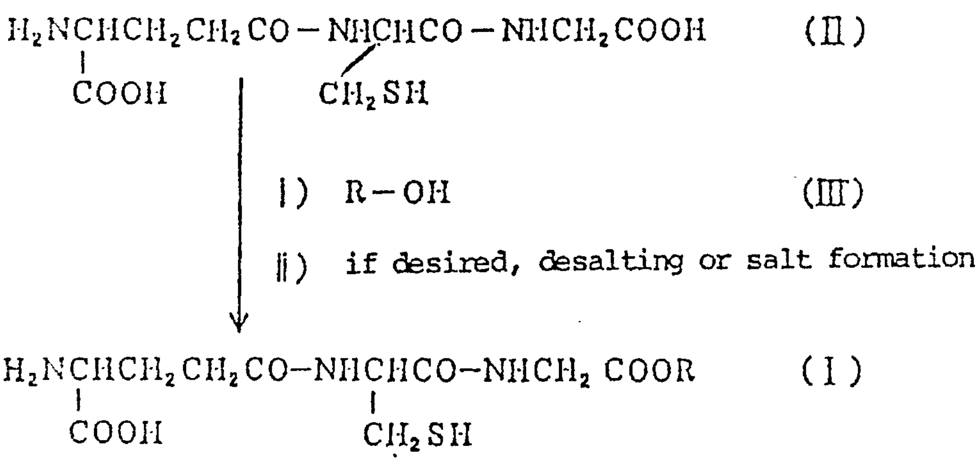


wherein R is as defined above.

The process can be carried out by reacting glutathione (II) with alcohol (III) in the presence of acid (for example, sulfuric acid) to produce glutathione monoalkyl ester salt, with desalting or salt conversion if desired.

Compounds for this invention are preferably administered in free form, but they could be pharmaceutically acceptable salts with inorganic acids such as hydrochlorides, nitrates, sulfates, etc. or with organic acids such as oxalates, p-toluene-sulfonates, maleates, etc. When the compound is in salt form it can, if necessary or desired, be first desalted or administered with addition of base such as sodium hydrogencarbonate that does not adversely affect the living body.

The glutathione monoalkyl esters (I) and salts thereof for the present invention can be prepared into tablets, powders, granulates, granules, capsules, pills, liquids, injections, etc. using conventional pharmaceutically acceptable carriers for oral or parenteral administration. Dose may be varied depending upon administration route, body weight, age, condition, etc. of the patient. For myocardial infarction administration may be by continuous i.v. injection from immediately after emergency admission into hospital over a period of one to two hours before, during and after the operation for reopening the occluded corononary artery. Depending on the seriousness, it may be preferable to combine this with an individual dose or doses. For an adult, individual doses are suitably from 500 to 5,000 mg, preferably 1,000 to 3,000 mg, each; and in the case of continuous administration, the dosage is suitably from 30 to 100 mg/min.

The isopropyl ester (I) and salts have acute toxicity ($LD_{50}$) of approximately 5.3 g/kg of intraperitoneal injection into mice.

Drugs comprising as an effective component glutathione monoalkyl esters (I) or salts thereof control disorders due to occluded coronary artery reperfusion in ischemic heart diseases and are useful as therapeutic agents for ischemic heart diseases.

Formulations of these therepeutic agents and their effect on ischemic heart diseases is illustrated in more detail in the Examples. The Reference Example shows the synthesis of monoisopropyl ester used in the Examples.

Example 1

Injection Formulation

Glutathione monoisopropyl ester sulfate was suspended in purified water in a concentration of 45 mg/ml and the suspension was cooled to 5° C or below. About 2-fold mols of sodium hydrogencarbonate were added to dissolve the ester sulfate. After adjusting to pH 4, the solution was diluted to a concentration of 40 mg/ followed by aseptic filtration; 25 ml.aliquots of the solution were charged into vials and freeze dried.

Example 2

Action on ischemic heart diseases

The inhibitory action of glutathione monoisopropyl ester on ventricular extra systole frequency when the occluded coronary artery was reperfused in dogs, was tested by the following method.

Method of measurement:

Measurement was carried out by partially modifying Gibsons' method (J. Cardiovasc. Pharmacol., 5, 517-524, 1983). Dogs (mongrel, female and male, 9-18 kg) were anesthetized with pentobarbital, the chest was opened under artificial respiration, and then the left coronary artery anterior descending segmental branch was occluded for 60 minutes. Thereafter, reperfusion was carried out for 20 minutes and then blood pressure and heart rate as well as the heart surface electrocardiogram were measured.

The glutathione monoisopropyl ester (GE) was intravenously administered in a single dose of 300 mg/kg 30 minutes after the coronary artery occlusion and and continuous injection was carried out in an amount of 10 mg/kg/min, i.v., until the completion of this test from 40 minutes after the coronary artery occlusion. The effect of an agent was evaluated by the ventricular extra systole frequency during the period of reperfusion.

Results

The action of GE on the ventricular extra systole frequency during the coronary artery occlusion/reperfusion is shown below.

| Drug | Number of animals | Ventricular extra systole frequency (counts/min) |
|---|---|---|
| Control group (group given physiological saline) | 13 | 35.9±10.2 |
| GE-treated group (300 mg/kg i.v. + 10 mg/kg/min. i.v.) | 9 | 10.0±5.6* |

The values in the Table represent the mean value ± the standard deviation.

* $P < 0.05$: This represents a significant difference as compared to the control group (Student's t-test.)

Reference Example

Production of glutathione monoisopropyl ester:

(1) To 800 ml of isopropyl alcohol was added 124 g of glutathione. While stirring, 42 ml of 95% sulfuric acid was added dropwise to the mixture. Heat evolved but it was unnecessary to cool. About one hour later, the system became a homogeneous solution and glutathione monoisopropyl ester (isopropyl r-L-glutamyl-L-cysteinyl glycinate) sulfate crystals began to precipitate about 24 hours after. The stirring was further continued overnight.
The crystals were taken by filtration, washed with 200 ml of isopropyl alcohol and dried under reduced pressure to give 88.5 g of the sulfate. A part of the crystals was recrystallized from water-isopropyl alcohol mixture (mixing ratio, 1:5) and purified to provide a sample for analysis.
Melting point: 145-150°C

Elemental analysis (as $C_{13}H_{23}N_3O_6S.1/2H_2SO_4,1/2H_2O$)

| | C | H | N | S |
|---|---|---|---|---|
| Calcd. (%) | 38.32 | 6.18 | 10.31 | 11.80 |
| Found (%) | 38.11 | 6.34 | 10.32 | 12.10 |

(2) In 1.2 liters of water was dissolved 50.0 g of the unpurified sulfate obtained in (1). The solution was charged into 1.5 liters of HP-20, eluted with water and then eluted with a methanol-water mixture (mixing ratio 1:1) to give 2.5 liters of the fraction containing the product. After concentrating the fraction, the concentrate was freeze dried to give 33.8 g of glutathione monoisopropyl ester.

(i) Melting point: 184-186°C
(ii) Infrared absorption spectra (KBr, $cm^{-1}$) 1730, 1635, 1525, 1400, 1370, 1205, 1100
(iii) Specific rotary power ($[\alpha]_D^{21}$) -31.0 (c=1.0, water)
(iv) Nuclear magnetic resonance spectra (DMSO-$d_6$, δppm) 1.20 (6H, d, J=6Hz), 1.72-2.16 (2H, m), 2.20-2.40 (2H, m), 2.64-2.86 (2H, m), 3.20-3.56 (1H, m), 3.80 (2H, m), 4.20-4.60 (1H, m), 4.68-5.08 (1H, m) be

**Claims**

1. The use for the preparation of therapeutic agent for ischemic heart diseases of an effective component selected from glutathione monoalky esters of formula (I) and salts thereof

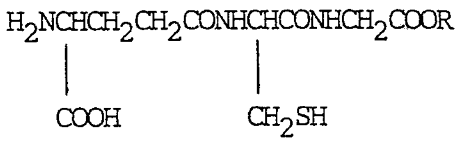

(I)

wherein R represents an alkyl group, preferably of 1-10 carbon atoms - e.g. an isopropyl group.

2. A therapeutic agent for ischemic heart diseases comprising an effective component selected from glutathione monoalkyl esters of formula (I) and salts thereof

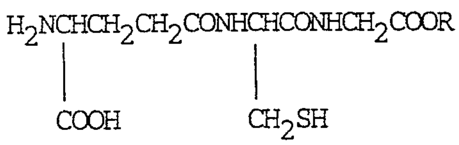

(I)

wherein R represents an alkyl group.